# EUROPÄISCHE PATENTSCHRIFT

(11) **EP 2 823 827 B1**
(45) Veröffentlichungstag und Bekanntmachung des Hinweises auf die Patenterteilung: **21.12.2016**
(21) Anmeldenummer: 14173059.8
(22) Anmeldetag: 18.06.2014
(51) Int. Cl.: A61L 2/22, A61L 2/14

(54) **Gerät zur Reinigung und Desinfektion von Steckbecken und Urinflaschen**
Device for cleaning and disinfecting bed-pans and urine bottles
Appareil de nettoyage et de désinfection pour bassins de lit et pistolets

(30) Priorität: 21.06.2013 DE 102013106513; 04.07.2013 DE 102013107017
(43) Veröffentlichungstag der Anmeldung: 14.01.2015
(73) Patentinhaber: BG Edelstahl und Kunststofftechnik für Krankenhaus Industrie und Wasserwirtschaft GmbH, 45739 Oer-Erkenschwick (DE)
(72) Erfinder: Barselak, Patrick, 45731 Waltrop (DE)
(74) Vertreter: Gesthuysen Patent- und Rechtsanwälte

(56) Entgegenhaltungen:
- EP-A1- 2 425 806
- CH-A5- 669 116
- DE-U1-202009 013 764
- US-A1- 2010 145 160
- US-B1- 6 431 189
- DATABASE WPI Week 201010 Thomson Scientific, London, GB; AN 2010-A92402 XP002732986, & KR 2009 0123297 A (E TND CO LTD) 2. Dezember 2009 (2009-12-02)

## Beschreibung

Die Erfindung betrifft ein Gerät zur Reinigung und Desinfektion von Steckbecken und Urinflaschen, mit einem Gerätegehäuse, einer Steuereinheit, einer Reinigungskammer und einer Klappe, wobei die Reinigungskammer mit der Klappe verschließbar ist.

Geräte zur Reinigung und Desinfektion von Steckbecken und Urinflaschen werden auch Steckbeckenspülgeräte genannt und sind im Stand der Technik in einer Vielzahl von Ausgestaltungen bekannt. In Krankenhäusern und Pflegeeinrichtungen werden derartige Geräte u. a. dazu eingesetzt, Steckbecken und Urinflaschen nach der Benutzung vollautomatisch zu reinigen und zu desinfizieren. Die zu reinigenden Behälter werden dazu in die Reinigungskammer des Gerätes eingebracht und ein Reinigungsprogramm gestartet. Reinigungsprogramme üblicher vollautomatischer Steckbeckenspülgeräte sehen vor, dass die verschmutzten Behälter zunächst mit Wasser vorgereinigt werden, damit Schmutzstoffe und Ablagerungen entfernt werden und in den Abfluss fließen können. Die Wasserführung erfolgt dabei nicht im Umlaufprinzip wie beispielsweise bei Haushaltsspülmaschinen, da aus hygienischen Gründen die hochgradig mit Bakterien verunreinigten Abwässer nicht erneut mit den Behältern in Kontakt geraten sollen. Anschließend wird, je nach Ausgestaltung des Geräts und der Art der zu reinigenden Behälter, eine thermische, chemische oder thermo-chemische Desinfektion der Behälter innerhalb der Reinigungskammer durchgeführt.

Aus der EP 2 425 806 A1 ist ein Verfahren zum Reinigen von Steckbecken, Urinflaschen und/oder dergleichen Spülgüter bekannt, bei dem eine mechanische Reinigung, anschließend eine chemische Reinigung und abschließend eine thermische Desinfektion durchgeführt wird. Der Verfahrensschritt der chemischen Reinigung wird dreistufig durchgeführt, wodurch erreicht wird, dass auf eine manuelle Nachreinigung möglichst verzichtet werden kann.

Beim Umgang mit benutzten Steckbecken und Urinflaschen können die Hände des Pflegepersonals mit unterschiedlichen Keimen, z. B. Bakterien und Viren, kontaminiert werden, so dass eine regelmäßige Desinfektion der Hände erforderlich ist, um insbesondere eine gesundheitliche Gefährdung des Pflegepersonals und der Patienten zu reduzieren. Nur durch eine regelmäßige Desinfektion der Hände des Pflegepersonals kann eine Ausbreitung von Keimen wirksam unterbunden werden, so dass eine wirksame Infektionsprävention gewährleistet ist.

Eine Herausforderung besteht im Allgemeinen allerdings darin, eine regelmäßige Desinfektion zumindest der Hände des Pflegepersonals in regelmäßigen Abständen tatsächlich sicherzustellen, da die Durchführung einer Desinfektion im Berufsalltag, insbesondere unter Zeitdruck, vergessen wird.

Der Erfindung liegt deshalb die Aufgabe zugrunde, ein Gerät zur Reinigung und Desinfektion von Steckbecken und Urinflaschen anzugeben, das zur Minimierung der Ausbreitung von Keimen durch einen Benutzer beiträgt.

Die vorgenannte Aufgabe ist gemäß der vorliegenden Erfindung zunächst und im Wesentlichen dadurch gelöst, dass mindestens eine Desinfektionseinheit zur Desinfektion der Hände eines Benutzers umfasst ist, und dass die Desinfektionseinheit mit der Steuereinheit verbunden ist. Die Steuereinheit ist vorzugsweise auch die Steuereinheit, die unter anderem den Ablauf der Reinigungsprogramme innerhalb der Reinigungskammer des Geräts steuert. Die Steuereinheit umfasst vorzugsweise einen Mikrocontroller oder Mikroprozessor, der die erforderlichen Abläufe steuert. Die Steuereinheit ist im Gerätegehäuse angeordnet.

Die Steuereinheit und die Desinfektionseinheit sind miteinander verbunden, vorzugsweise derart miteinander verknüpft, dass zumindest Signale von der Desinfektionseinheit in Richtung der Steuereinheit übertragen werden können - unidirektional -, bevorzugt aber sowohl von Desinfektionseinheit auf die Steuereinheit als auch umgekehrt - bidirektional. Die Verbindung ist vorteilhaft als elektrische Verbindung ausgebildet. Die Verknüpfung zwischen Desinfektionseinheit und Steuereinheit ist bevorzugt derart technisch realisiert, dass die Steuereinheit über eine Benutzung der Desinfektionseinheit "informiert" wird. Diese Information erfolgt beispielsweise durch ein Freigabesignal von der Desinfektionseinheit, das bei oder unmittelbar nach einer Benutzung der Desinfektionseinheit, also beispielsweise einer Entnahme eines Desinfektionsmittels, an die Steuereinheit übermittelt wird. Die Desinfektionseinheit übermittelt das Freigabesignal folglich aktiv. Dieses Freigabesignal kann beispielsweise durch einen einfacher Taster realisiert werden, der bei einer Benutzung der Desinfektionseinheit automatisch betätigt wird und somit ein Schaltsignal an die Steuereinheit liefert.

Alternativ dazu ist auch vorgesehen, dass die Steuereinheit die Desinfektionseinheit überwacht, wodurch eine Benutzung der Desinfektionseinheit erkannt wird.

Die Steuereinheit ist mit der Desinfektionseinheit verbunden und ist folglich derart ausgebildet und eingerichtet, dass in der Steuereinheit die Information verarbeitet wird, ob die Desinfektionseinheit benutzt worden ist. Diese Information wird vorzugsweise dazu verwendet, den Benutzer auf eine Benutzung der Desinfektionseinheit mit einem akustischen und/oder optischen Signal hinzuweisen. Ein optisches Signal wird beispielsweise in einer Anzeigeinrichtung angezeigt, die am Gerätegehäuse angeordnet ist.

Die Steuereinheit ist beispielsweise derart eingerichtet und ausgebildet, dass eine optische und/oder akustische Erinnerung des Benutzers erfolgt, wenn die Klappe geschlossen und ein Reinigungsprogramm ausgewählt worden ist. Bevor der Benutzer dann das Gerät verlässt, wird er zur Desinfektion der Hände aufgefordert. Das akustische und/oder optische Signal erfolgt dann, wenn das Reinigungsprogramm startet, aber in der Steuereinheit noch kein Freigabesignal von der Desinfektionseinheit vorliegt. Insbesondere kann diese Erinnerung auch zeitlich verzögert erfolgen, beispielsweise zwischen fünf und zehn Sekunden nach dem Start des Reinigungsprogramms. Wird die Desinfektionseinheit nach Beginn des Reinigungsprogramms oder nach Schließen der Klappe genutzt, erfolgt kein Signal. Wird die Desinfektionseinheit benutzt, nachdem ein Signal erfolgt ist, wird das Signal durch das Freigabesignal der Desinfektionseinheit an die Steuereinheit gelöscht.

Ferner ist auch vorgesehen, dass die Steuereinheit derart eingerichtet und ausgebildet ist, dass die Steuereinheit den Benutzer mit einem vorgenannten Signal an eine Nutzung der Desinfektionseinheit erinnert, unmittelbar nachdem ein Reinigungsprogramm beendet ist oder dann, wenn die Klappe nach abgeschlossenem Reinigungsprogramm geöffnet wird.

Aus dem Stand der Technik sind Desinfektionsvorrichtungen insbesondere zur Desinfektion der Hände beispielsweise aus der US 2010/0145160 A1 bekannt.

Insbesondere um eine Desinfektion der Hände durch einen Benutzer einfacher zu gestalten, ist gemäß einer ersten Ausgestaltung vorgesehen, dass die Desinfektionseinheit als Desinfektionsmittelspender, als Sprühkammer oder als System zur Desinfektion mit Plasma ausgebildet ist. Die Desinfektionseinheit dient zur Desinfektion der Hände des Benutzers nach dem Einbringen der zu reinigenden Behälter in das Gerät und/oder vor dem Öffnen des Geräts nach erfolgter Reinigung. Eine Desinfektion der Hände erfolgt dabei entweder chemisch, also mit einem Desinfektionsmittel, oder nach einer neuartigen Methode mit kaltem Plasma.

Die Desinfektionseinheit ist dazu beispielsweise als Desinfektionsmittelspender ausgebildet, der eine bestimmte Menge eines Desinfektionsmittels auf eine Hand oder nacheinander auf beide Hände aufgibt, damit der Benutzer anschließend durch Verreiben des Desinfektionsmittel eine gleichmäßige Desinfektion der Hände vornehmen kann. Das Desinfektionsmittel kann mit dem Desinfektionsmittelspender beispielsweise auch aufgesprüht werden. Alternativ dazu ist die Desinfektionseinheit als Sprühkammer ausgebildet, in die der Benutzer beide Hände für eine vorbestimmte Zeit eingibt, wobei die Hände durch Versprühen eines Desinfektionsmittels innerhalb der Sprühkammer desinfiziert werden.

Ein neuartiges System zur Desinfektion von Hautpartien eines Benutzers ist eine sogenannte Desinfektion mit kaltem Plasma. Eine Desinfektion erfolgt durch Plasmaanwendung auf die zu desinfizierenden Hautpartien, hier insbesondere auf die zu desinfizierenden Hände. Das Plasma wir dazu mittels Düsen auf die desinfizierenden Hautbereiche aufgebracht, wobei die ionisierte Luft eine Temperatur aufweist, die für die menschliche Haut unschädlich ist. Die Verwendung von kaltem Plasma hat den Vorteil, dass innerhalb von weniger Sekunden sämtliche Keime auch unterhalb der Fingernägel und der Hautfalten beseitigt werden.

Insbesondere um das Risiko einer unterlassenen Desinfektion der Hände zusätzlich zu reduzieren, ist gemäß einer weiteren Ausgestaltung vorgesehen, dass das Gerät zur Reinigung und Desinfektion, insbesondere die Steuereinheit, derart eingerichtet und ausgebildet ist, dass der Ablauf eines Reinigungsprogramms in der Reinigungskammer erst freigegeben wird, nachdem eine Benutzung der Desinfektionseinheit erfolgt ist, insbesondere dass eine Benutzung der Desinfektionseinheit nach dem Schließen der Klappe erfolgt ist. Die Steuereinheit überwacht folglich entweder aktiv die Desinfektionseinheit oder erhält von der Desinfektionseinheit ein Freigabesignal, das die Information übermittelt, dass eine Benutzung der Desinfektionseinheit erfolgt ist. Sobald die Steuereinheit über diese Information verfügt, wird entweder das Reinigungsprogramm in der Reinigungskammer unmittelbar von der Steuereinheit gestartet oder ein Start des Reinigungsprogramms wird durch die Steuereinheit freigegeben, so dass dieser der Benutzer das Reinigungsprogramm, beispielsweise durch Knopfdruck, starten kann.

Dadurch, dass vor Ablauf des Reinigungsprogramms in der Reinigungskammer eine Benutzung der Desinfektionseinheit erfolgen muss, ist sichergestellt, dass der Benutzer, der beim Beladen des Geräts mit den zu reinigenden und kontaminierten Behältern in Kontakt gekommen ist, nach dem Beladen des Geräts eine Desinfektion der Hände vornimmt. Andernfalls ist eine Benutzung des Geräts und damit eine Reinigung der kontaminierten Behälter nicht möglich.

Besonders bevorzugt ist vorgesehen, dass die Steuereinheit derart eingerichtet und ausgebildet ist, dass eine Freigabe des Ablaufs des Reinigungsprogramms dann erfolgt, wenn eine Benutzung der Desinfektionseinheit nach dem Schließen der Klappe erfolgt ist. Es hat sich als vorteilhaft herausgestellt, wenn der Benutzer ein Reinigungsprogramm auswählt und dann die Klappe schließt bzw. die Klappe schließt und dann ein Reinigungsprogramm auswählt und anschließend die Desinfektionseinheit benutzt, wobei die Benutzung der Desinfektionseinheit dann zu einem automatischen Start des Reinigungsprogramms führt. Auf diese Weise kommt der Benutzer nach der Desinfektion der Hände nicht mehr mit dem Gerät in Kontakt, wodurch eine erneute Kontamination zuverlässig unterbunden wird. Die Steuereinheit ist folglich derart eingerichtet und ausgebildet, dass ein Reinigungsprogramm nicht startet, wenn nicht nach der Programmwahl eine Benutzung der Desinfektionseinheit erfolgt. Eine Benutzung der Desinfektionseinheit wird beispielsweise durch die Entnahme von Desinfektionsmittel aus einem Desinfektionsmittelspender realisiert.

Gemäß einer weiteren bevorzugten Weiterbildung ist vorgesehen, dass das Gerät, insbesondere die Steuereinheit, derart eingerichtet und ausgebildet ist, dass eine Freigabe der Klappe nach abgeschlossenem Reinigungsprogramm erst erfolgt, nachdem eine Benutzung der Desinfektionseinheit erfolgt ist. Die Steuereinheit bzw. das Gerät, ist derart eingerichtet und ausgebildet, dass ein Öffnen der Klappe zur Entnahme der gereinigten und desinfizierten Behälter erst dann möglich ist, wenn vorab eine Benutzung der Desinfektionseinheit, insbesondere eine Entnahme von Desinfektionsmittel, erfolgt ist.

An der Klappe ist dazu beispielsweise eine Verriegelungsvorrichtung vorgesehen, die häufig ohnehin vorhanden ist, um ein Öffnen der Klappe während des Betriebs des Geräts zu verhindern. Die Steuereinheit sorgt dafür, dass eine Freigabe der Klappe, also eine Freigabe der Verriegelungsvorrichtung, nach erfolgter Reinigung und Desinfektion der in der Reinigungskammer vorhandenen Behälter erst dann erfolgt, wenn vorab eine Benutzung der Desinfektionseinheit erfolgt ist, folglich die Hände des Benutzers desinfiziert sind und ein Freigabesignal von der Desinfektionseinheit vorliegt. Dadurch wird eine erneute Kontamination der gereinigten Behälter durch die Hände des Benutzers verhindert.

Besonders bevorzugt ist vorgesehen, dass das Gerät, insbesondere die Steuereinheit, derart eingerichtet und ausgebildet ist, dass sowohl der Ablauf des Reinigungsprogramms in der Reinigungskammer erst freigegeben wird, nachdem eine Benutzung der Desinfektionseinheit erfolgt ist, als auch eine Freigabe der Klappe nach abgeschlossenem Reinigungsprogramm erst dann erfolgt, nachdem eine Benutzung der Desinfektionseinheit erfolgt ist. Durch eine derartige Ausgestaltung wird sichergestellt, dass ein Benutzer sich nach dem Einräumen der kontaminierten Behälter die Hände desinfiziert als auch, dass der Benutzer seine Hände desinfiziert bevor er die gereinigten Behälter aus der Reinigungskammer des Geräts entnimmt.

Vorzugsweise ist das Gerät, insbesondere die Steuereinheit, derart eingerichtet und ausgebildet, dass durch eine Benutzung der Desinfektionseinheit ein automatischen Schließen der Klappe nach dem Beladen der Reinigungskammer und/oder ein automatisches Öffnen der Klappe nach durchlaufenem Reinigungsprogramm erfolgt. Vorteilhaft bewirkt eine Benutzung der Desinfektionseinheit auch einen automatischen Start eines Reinigungsprogramms. Durch eine derartige Ausgestaltung wird Anzahl der erforderlichen Berührungen des Geräts durch den Benutzer reduziert.

Gemäß einer bevorzugten Ausgestaltung, die sich insbesondere bei einer Desinfektionseinheit als vorteilhaft herausgestellt hat, die als Desinfektionsmittelspender ausgebildet ist, ist vorgesehen, dass eine Freigabe der Klappe nach der Benutzung der Desinfektionseinheit erst nach einer vorbestimmten Einwirkzeit erfolgt. Dazu ist vorgesehen, dass der Benutzer die Desinfektionseinheit benutzt und erst nach der vorbestimmten Einwirkzeit ein Öffnen der Klappe zur Entnahme der gereinigten Behälter möglich ist, nämlich durch die Steuereinheit freigegeben wird. Eine besonders bevorzugte Einwirkzeit sind dabei 30 Sekunden, innerhalb der übliche Desinfektionsmittel ihre volle Wirkung entfalten.

Um den Kontakt des Benutzers mit dem Gerät, insbesondere mit kontaminierten Händen, zu verringern, ist gemäß einer weiteren Ausgestaltung vorgesehen, dass die Desinfektionseinheit mindestens einen Sensor umfasst, und dass mit dem Sensor zumindest die Anwesenheit mindestens einer Hand eines Benutzers erkennbar ist. Sobald der Sensor die Anwesenheit einer Hand erkennt, wird beispielsweise ein Desinfektionsmittel ausgegeben, ausgesprüht oder die Desinfektion innerhalb einer Sprühkammer oder eines Systems zur Plasmadesinfektion gestartet. Durch den Sensor braucht der Benutzer die Desinfektionseinheit zum Start der Desinfektion nicht berühren.

Ein Ausgangssignal des Sensors kann beispielsweise auch als Eingangssignal für die Steuereinheit verwendet werden, nämlich als Freigabessignal, das die Information übermittelt bzw. als solche interpretiert wird, dass eine Benutzung der Desinfektionseinheit erfolgt ist. Als Sensor sind beispielsweise Bewegungssensoren, Wärmesensoren oder auch eine Kamera geeignet. Eine Kamera ist beispielsweise dann besonders vorteilhaft, wenn ein Verreiben eines Desinfektionsmittels auf den Händen überwacht werden soll, beispielsweise um ein Bewegungsmuster innerhalb einer Einwirkzeit zu erfassen. Die Hände müssen dazu vorzugsweise kontinuierlich innerhalb eines Zeitraums von 30 Sekunden im Erkennungsbereich der Kamera bewegt werden, um eine Freigabe des Reinigungsprogramms oder ein Öffnen der Klappe zu ermöglichen.

Vorzugsweise um ein kompaktes Gerät zu realisieren, ist gemäß einer weiteren Ausgestaltung vorgesehen, dass die Desinfektionseinheit zumindest teilweise in das Gerätegehäuse eingelassen ist. Je nach Ausgestaltung der Desinfektionseinheit ist aber auch vorgesehen, dass die Desinfektionseinheit an einer Außenseite des Gerätegehäuses vorgesehen ist. Ein Einlassen der Desinfektionseinheit hat sich aber bei allen Varianten der Desinfektionseinheit, insbesondere bei einem Desinfektionsmittelspender und bei einer Sprühkammer als vorteilhaft herausgestellt.

Insbesondere um den Verdrahtungsaufwand innerhalb des Gerätegehäuses zu verringern, aber auch um ein voneinander beabstandetes Betreiben des Geräts und der Desinfektionseinheit zu ermöglichen, ist gemäß einer weiteren Ausgestaltung vorgesehen, dass zwischen der Desinfektionseinheit und der Steuereinheit eine Funkverbindung ausgebildet ist. Diese ermöglicht, dass die Desinfektionseinheit zusammen mit der Steuereinheit innerhalb des Gerätegehäuses angeordnet sind, aber gleichwohl eine Datenübertragung zwischen der Desinfektionseinheit und der Steuereinheit über eine Funkverbindung realisiert ist.

Besonders vorteilhaft ist diese Ausgestaltung aber dann, wenn die Desinfektionseinheit beabstandet vom Gerätegehäuse des Geräts angeordnet ist, beispielsweise im Bereich der Tür des Raumes, in dem das Gerät angeordnet ist. So kann das Reinigungsprogramm durch eine Desinfektion der Hände beim Verlassen des Raumes automatisch gestartet werden bzw. ein Öffnen der Klappe automatisch nach Betreten des Raumes und nach erfolgter Desinfektion freigegeben werden, so dass der Weg, den der Benutzer zwischen der Benutzung der Desinfektionseinheit und dem Öffnen der Klappe zurücklegt, für die Einwirkzeit ausreicht. Das eine Funkverbindung "ausgebildet" ist, bedeutet dabei lediglich, dass ein Signalaustausch per Funk - auch bedarfsweise - möglich ist; eine dauerhafte Funkverbindung ist dazu nicht erforderlich. Die Funkverbindung ist beispielweise mittels WirelessLAN, Bluetooth oder anderer Funkstandards, die insbesondere für den Nahbereich geeignet sind, realisiert.

Die erfindungsgemäße Aufgabe wird ferner durch ein Verfahren zum Betrieb eines Geräts zur Reinigung und Desinfektion von Steckbecken und Urinflaschen, mit einem Gerätegehäuse, einer Steuereinheit, einer Reinigungskammer und einer Klappe, wobei die Reinigungskammer mit der Klappe verschließbar ist, wobei mindestens eine Desinfektionseinheit zur Desinfektion der Hände eines Benutzers umfasst ist, und wobei die Desinfektionseinheit mit der Steuereinheit verbunden ist, dadurch gelöst, dass eine Generierung eines Freigabesignals durch Benutzung der Desinfektionseinheit und Übermittlung des Freigabesignals an die Steuereinheit erfolgt. Nachfolgend erfolg eine Berücksichtigung des Freigabesignals durch die Steuereinheit bei der Steuerung des Betriebsablaufs des Geräts. Diese Berücksichtung kann beispielsweise dadurch erfolgen, dass zu einem definierten Zeitpunkt des Betriebsablaufs, z. B. unmittelbar nach dem Start eines Reinigungsprogramms ein Signal angezeigt wird, dass eine Desinfektion der Hände zu erfolgen hat. Ferner kann das Freigabesignal nach erfolgter Reinigung berücksichtigt werden, beispielsweise dass ein Signal angezeigt wird, wenn die Klappe geöffnet wird, aber noch kein Freigabesignal von der Desinfektionseinheit vorliegt.

Gemäß einer Ausgestaltung des Verfahrens ist vorgesehen, dass eine Freigabe des Ablaufs des Reinigungsprogramms durch die Steuereinheit erst erfolgt, wenn nach dem Schließen der Klappe ein Freigabesignal übermittelt wird. Der Ablauf des Reinigungsprogramms wird durch die Steuereinheit folglich nach dem Schließen der Klappe und der Auswahl des Reinigungsprogramms so lange blockiert, bis ein Freigabesignal von der Desinfektionseinheit übermittelt wird, nämlich eine Benutzung der Desinfektionseinheit durch einen Benutzer erfolgt ist.

Alternativ oder ergänzend, ist gemäß einer weiteren Ausgestaltung des Verfahrens vorgesehen, dass eine Freigabe der Klappe erst erfolgt, wenn nach abgeschlossenem Reinigungsprogramm ein Freigabesignal übermittelt wird. Das Öffnen der Klappe zum Ausräumen der gereinigten Behälter wird folglich von der Steuereinheit so lange blockiert, bis ein Freigabesignal von der Desinfektionseinheit übermittelt worden ist, also eine Benutzung der Desinfektionseinheit erfolgt ist. Die Übermittlung des Freigabesignals muss dazu nach Abschluss des Reinigungsprogramms erfolgen.

Im Einzelnen gibt es nun eine Vielzahl von Möglichkeiten, das erfindungsgemäße Gerät und das Verfahren auszugestalten und weiterzubilden. Dazu wird verwiesen sowohl auf die den Patentansprüchen 1 und 9 nachgeordneten Patentansprüche als auch auf die nachfolgende Beschreibung von bevorzugten Ausführungsbeispielen in Verbindung mit der Zeichnung. In der Zeichnung zeigen:
- Fig. 1: ein Ausführungsbeispiel eines erfindungsgemäßen Geräts in perspektivischer Ansicht,
- Fig. 2: das Ausführungsbeispiel eines erfindungsgemäßen Geräts gemäß Fig. 1, und
- Fig. 3: ein Ausführungsbeispiel eines erfindungsgemäßen Geräts in Vorderansicht.

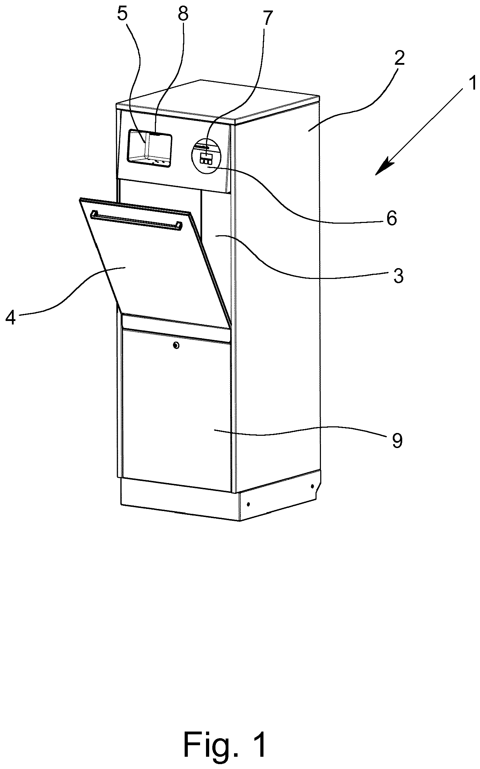

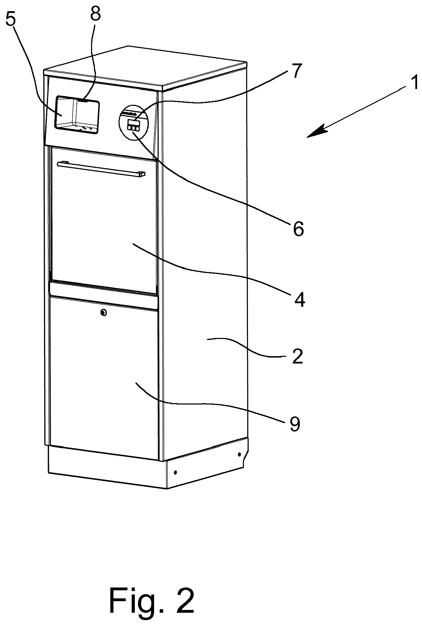

Fig. 1 zeigt ein Gerät 1 zur Reinigung und Desinfektion von Steckbecken und Urinflaschen. Das Gerät 1 umfasst ein Gerätegehäuse 2, eine - nicht dargestellte - Steuereinheit, eine Reinigungskammer 3 und eine Klappe 4. Gemäß Fig. 1 ist die Klappe 4 teilweise geöffnet. Fig. 2 zeigt das Ausführungsbeispiel eines Geräts zur Reinigung und Desinfektion von Steckbecken und Urinflaschen gemäß Fig. 1 mit geschlossener Klappe 4. Die Reinigungskammer 3 ist mit der Klappe 4 verschließbar und ist innerhalb des Gerätegehäuses 2 angeordnet.

Fig. 1 und Fig. 2 zeigen, dass das Gerät 1 eine Desinfektionseinheit 5 zur Desinfektion der Hände eines Benutzers umfasst. Die Desinfektionseinheit 5 ist bei diesem Ausführungsbeispiel als Desinfektionsmittelspender ausgebildet und ist vollständig in das Gerätegehäuse eingelassen. Die als Desinfektionsmittelspender ausgebildete Desinfektionseinheit 5 verfügt über eine Düse 8, mit der ein Desinfektionsmittel auf die Hände eines Benutzers aufgebbar ist. Die Desinfektionseinheit 5 oberhalb der Klappe 4 in dem Gerätegehäuse 2 angeordnet und ist mit der Steuereinheit des Geräts 1 verknüpft. Es erfolgt folglich eine Übermittlung von Steuersignalen mindestens von der Desinfektionseinheit 5 in Richtung der Steuereinheit. Die Steuereinheit ist ebenfalls innerhalb des Gerätegehäuses 2 angeordnet und dient u. a. zur Ablaufsteuerung der Reinigungsprogramme innerhalb der Reinigungskammer 3.

Das Gerät 1 gemäß dem Ausführungsbeispiel der Fig. 1 und 2, insbesondere die Steuereinheit des Geräts 1, ist derart eingerichtet und ausgebildet, dass ein Ablauf eines Reinigungsprogramms in der Reinigungskammer 3 erst freigegeben wird, nachdem eine Benutzung der Desinfektionseinheit 5 durch einen Benutzer erfolgt ist.

Die Bedienung des Geräts 1 durch einen Benutzer, d. h. beispielsweise das Ein- und Ausschalten sowie die Wahl, der Start und das Beenden von Reinigungsprogrammen erfolgt über ein Bedienfeld 6, das auch über eine Anzeigeeinrichtung 7 verfügt. In der Anzeigeeinrichtung 7 werden dem Benutzer Informationen bezüglich der Programmwahl und weitere das Gerät 1 betreffende Informationen und Signale angezeigt.

Zur Reinigung und Desinfektion von Steckbecken und Urinflaschen sowie weiterer zu reinigender Behälter öffnet ein Benutzer die Klappe 4 des Gerätes 1 und räumt die zu reinigenden Behälter in die Reinigungskammer 3 des Geräts 1 ein. Anschließend schließt der Benutzer die Klappe 4 und wählt über das Bedienfeld 6 das gewünschte Reinigungsprogramm aus. Nachfolgend muss der Benutzer die Desinfektionseinheit 5 benutzen, indem mittels der Düse 8 ein Desinfektionsmittel auf die Hände des Benutzers aufgegeben wird. Anschließend ist durch die Benutzung der Desinfektionseinheit 5 der Ablauf des Reinigungsprogramms in der Reinigungskammer 3 freigegeben, so dass das Reinigungsprogramm beginnt. Benutzt der Benutzer die Desinfektionseinheit 5 nicht, startet das Reinigungsprogramm nicht. Dadurch ist sichergestellt, dass nach der Benutzung eine Desinfektion der Hände erfolgt.

Das Gerät 1 gemäß dem Ausführungsbeispiel der Fig. 1 und Fig. 2, insbesondere die Steuereinheit, ist zusätzlich derart eingerichtet und ausgebildet, dass nach durchlaufendem und abgeschlossenem Reinigungs- bzw. Desinfektionsprogramm ein Öffnen der Klappe 4 zum Ausräumen der gereinigten Behälter erst dann möglich ist, nachdem erneut eine Benutzung der Desinfektionseinheit 5 erfolgt ist. Auf diese Weise wird sichergestellt, dass die gereinigten Behälter nicht erneut durch die Hände des Benutzers kontaminiert werden.

Gemäß Fig. 1 und 2 ist in dem Gerätegehäuse 2 unterhalb der Klappe 4 zusätzlich ein Geräteschrank 9 vorgesehen, in dem Zubehör, beispielsweise Reinigungsmittel und Desinfektionsmittel, gelagert werden kann. Der Geräteschrank 9 ist ebenfalls mit einer Klappe verschlossen.

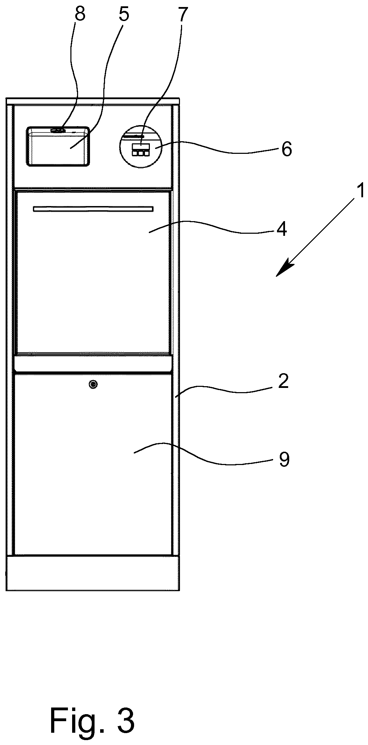

Fig. 3 zeigt Ausführungsbeispiel eines Geräts 1 zur Reinigung und Desinfektion von Steckbecken und Urinflaschen in Vorderansicht. Das Gerät 1 umfasst ebenfalls ein Gerätegehäuse 2, eine Steuereinheit, eine Reinigungskammer 3 und eine Klappe 4. Die Steuereinheit ist derart eingerichtet und ausgebildet, dass nach abgeschlossener Reinigung der zu reinigenden Behälter in der Reinigungskammer 3 eine Freigabe der Klappe 4 erst erfolgt, nachdem der Benutzer die Desinfektionseinheit 5 benutzt hat, die als Desinfektionsmittelspender mit einer Düse 8 ausgebildet ist. Der Benutzer muss folglich erst Desinfektionsmittel aus der Desinfektionseinheit entnehmen, um die Klappe 4 zum Ausräumen der Behälter öffnen zu können. Eine Bedienung des Geräts 1 erfolgt über das Bedienfeld 6, das eine Anzeigeinrichtung 7 umfasst.

## Patentansprüche

1. Gerät (1) zur Reinigung und Desinfektion von Steckbecken und Urinflaschen, mit einem Gerätegehäuse (2), einer Steuereinheit, einer Reinigungskammer (3) und einer Klappe (4), wobei die Reinigungskammer (3) mit der Klappe (4) verschließbar ist,
**dadurch gekennzeichnet,**
**dass** mindestens eine Desinfektionseinheit (5) zur Desinfektion der Hände eines Benutzers umfasst ist, und dass die Desinfektionseinheit (5) mit der Steuereinheit verbunden ist.

2. Gerät (1) nach Anspruch 1, **dadurch gekennzeichnet, dass** die Desinfektionseinheit (5) als Desinfektionsmittelspender, als Sprühkammer oder als System zur Desinfektion mit Plasma ausgebildet ist.

3. Gerät (1) nach Anspruch 1 oder 2, **dadurch gekennzeichnet, dass** die Steuereinheit derart eingerichtet und ausgebildet ist, dass der Ablauf eines Reinigungsprogramms in der Reinigungskammer (3) erst freigegeben wird, nachdem eine Benutzung der Desinfektionseinheit (5) erfolgt ist, insbesondere dass eine Benutzung der Desinfektionseinheit (5) nach dem Schließen der Klappe (4) erfolgt ist.

4. Gerät (1) nach einem der Ansprüche 1 bis 3, **dadurch gekennzeichnet, dass** die Steuereinheit derart eingerichtet und ausgebildet ist, dass eine Freigabe der Klappe (4) nach abgeschlossenem Reinigungsprogramm erst erfolgt, nachdem eine Benutzung der Desinfektionseinheit (5) erfolgt ist.

5. Gerät (1) nach Anspruch 4, **dadurch gekennzeichnet, dass** eine Freigabe der Klappe (4) nach der Benutzung der Desinfektionseinheit (5) erst nach einer vorbestimmten Einwirkzeit erfolgt, insbesondere einer Einwirkzeit von 30 Sekunden.

6. Gerät (1) nach einem der Ansprüche 1 bis 5, **dadurch gekennzeichnet, dass** die Desinfektionseinheit (5) mindestens einen Sensor umfasst, und dass mit dem Sensor zumindest die Anwesenheit mindestens einer Hand eines Benutzers erkennbar ist.

7. Gerät (1) nach einem der Ansprüche 1 bis 6, **dadurch gekennzeichnet, dass** die Desinfektionseinheit (5) zumindest teilweise in das Gerätegehäuse (2) eingelassen ist.

8. Gerät (1) nach einem der Ansprüche 1 bis 7, **dadurch gekennzeichnet, dass** zwischen der Desinfektionseinheit (5) und der Steuereinheit eine Funkverbindung ausgebildet ist.

9. Verfahren zum Betrieb eines Geräts (1) zur Reinigung und Desinfektion von Steckbecken und Urinflaschen, mit einem Gerätegehäuse (2), einer Steuereinheit, einer Reinigungskammer (3) und einer Klappe (4), wobei die Reinigungskammer (3) mit der Klappe (4) verschließbar ist, wobei mindestens eine Desinfektionseinheit (5) zur Desinfektion der Hände eines Benutzers umfasst ist, und wobei die Desinfektionseinheit (5) mit der Steuereinheit verbunden ist, nach einem der Ansprüche 2 bis 8, **gekennzeichnet durch** die folgenden Verfahrensschritte:
Generierung eines Freigabesignals **durch** Benutzung der Desinfektionseinheit (5) und Übermittlung des Freigabesignals an die Steuereinheit,
Berücksichtigung des Freigabesignals **durch** die Steuereinheit bei der Steuerung des Betriebsablaufs des Geräts (1).

10. Verfahren nach Anspruch 9, **dadurch gekennzeichnet, dass** eine Freigabe des Ablaufs des Reinigungsprogramms durch die Steuereinheit erst erfolgt, wenn nach dem Schließen der Klappe (4) ein Freigabesignal übermittelt wird.

11. Verfahren nach Anspruch 9 oder 10, **dadurch gekennzeichnet, dass** eine Freigabe der Klappe (4) erst erfolgt, wenn nach abgeschlossenem Reinigungsprogramm ein Freigabesignal übermittelt wird.

## Claims

1. Device (1) for cleaning and disinfecting of bedpans and urine bottles, having a device housing (2), a control unit, a cleaning chamber (3) and a flap (4), wherein the cleaning chamber (3) can be closed with the flap (4), **characterized in**
**that** at least one disinfection unit (5) for disinfecting a user's hands is comprised, and that the disinfection unit (5) is connected to the control unit.

2. Device (1) according to claim 1, **characterized in that** the disinfection unit (5) is designed as a disinfectant dispenser, as a spray chamber or as a system for disinfection using plasma.

3. Device (1) according to claim 1 or 2, **characterized in that** the control unit is equipped and designed so that clearance for running a cleaning program in the cleaning chamber (3) first takes place after the disinfection unit (5) has been used, in particular that the disinfection unit (5) is used after closing the flap (4).

4. Device (1) according to any one of claims 1 to 3, **characterized in that** the control unit is equipped and designed so that, after the cleaning program, clearance for opening the flap (4) first takes place after the disinfection unit (5) has been used.

5. Device (1) according to claim 4, **characterized in that** clearance for opening the flap (4) after using the disinfection unit (5) first takes place after a predetermined acting time, in particular an acting time of 30 seconds.

6. Device (1) according to any one of claims 1 to 5, **characterized in that** the disinfection unit (5) comprises at least one sensor, and that the presence of at least one user's hand can be recognized with the sensor.

7. Device (1) according to any one of claims 1 to 6, **characterized in that** the disinfection unit (5) is at least partially embedded in the device housing (2).

8. Device (1) according to any one of claims 1 to 7, **characterized in that** a wireless connection is formed between the disinfection unit (5) and the control unit.

9. Method for operating a device (1) for cleaning and disinfecting of bedpans and urine bottles, having a device housing (2), a control unit, a cleaning chamber (3) and a flap (4), wherein the cleaning chamber (3) can be closed with the flap (4), wherein at least one disinfection unit (5) for disinfecting a user's hands is comprised, and wherein the disinfection unit (5) is connected to the control unit according to any one of claims 2 to 8, **characterized by** the following method steps:
generating a clearance signal by using the disinfection unit (5) and transmitting the clearance signal to the control unit,
the control unit taking the clearance signal into account when controlling operation of the device (1).

10. Method according to claim 9, **characterized in that** clearance for running the cleaning program from the control unit first takes place after the flap (4) has been closed and a clearance signal has been transmitted.

11. Method according to claim 9 or 10, **characterized in that** clearance for opening the flap (4) first takes place when the cleaning program has finished and a clearance signal has been transmitted.

## Revendications

1. Appareil (1) de nettoyage et de désinfection de bassins de lit et de flacons d'urine, comprenant un boîtier d'appareil (2), une unité de commande, une chambre de nettoyage (3) et un volet (4), dans lequel la chambre de nettoyage (3) peut être fermée au moyen du volet (4),
**caractérisé en ce qu'**il est prévu au moins une unité de désinfection (5) pour désinfecter les mains d'un utilisateur et **en ce que** l'unité de désinfection (5) est reliée à l'unité de commande.

2. Appareil (1) selon la revendication 1, **caractérisé en ce que** l'unité de désinfection (1) est réalisée sous la forme d'un distributeur d'agent de désinfection, sous la forme d'une chambre de pulvérisation ou sous la forme d'un système de désinfection au plasma.

3. Appareil (1) selon la revendication 1 ou 2, **caractérisé en ce que** l'unité de commande est conçue et configurée de manière à ce que l'exécution d'un programme de nettoyage soit tout d'abord déclenché dans la chambre de nettoyage (3), puis qu'une utilisation de l'unité de désinfection (5) soit effectuée, notamment de manière à ce qu'une utilisation de l'unité de désinfection (5) soit effectuée après la fermeture du volet (4).

4. Appareil (1) selon l'une quelconque des revendications 1 à 3, **caractérisé en ce que** l'unité de commande est conçue et configurée de manière à ce qu'un déclenchement du volet (4) ne soit effectué qu'après la fin du programme de nettoyage, et qu'une utilisation de l'unité de désinfection (5) soit ensuite effectuée.

5. Appareil (1) selon la revendication 4, **caractérisé en ce qu'**un déclenchement du volet (4) est effectué après l'utilisation de l'unité de désinfection (5) immédiatement après un temps de mise en oeuvre prédéterminé, notamment un temps d'action de 30 secondes.

6. Appareil (1) selon l'une quelconque des revendications 1 à 5, **caractérisé en ce que** l'unité de désinfection (5) comprend au moins un capteur et **en ce qu'**au moins la présence d'au moins une main d'un utilisateur peut être détectée au moyen du capteur.

7. Appareil (1) selon l'une quelconque des revendications 1 à 6, **caractérisé en ce que** l'unité de désinfection (5) est au moins partiellement engagée dans le boîtier d'appareil (2).

8. Appareil (1) selon l'une quelconque des revendications 1 à 7, **caractérisé en ce qu'**une liaison radio est établie entre l'unité de désinfection (5) et l'unité de commande.

9. Procédé de mise en fonctionnement d'un appareil (1) de nettoyage et de désinfection de bassins de lit et de flacons d'urine, comprenant un boîtier d'appareil (2), une unité de commande, une chambre de nettoyage (3) et un volet (4), dans lequel la chambre de nettoyage (3) peut être fermée au moyen du volet (4), dans lequel il est prévu au moins une unité de désinfection (5) pour désinfecter les mains d'un utilisateur et dans lequel l'unité de désinfection (5) est reliée à l'unité de commande, selon l'une quelconque des revendications 2 à 8, **caractérisé par** les étapes de procédé consistant à :
générer un signal de déclenchement par utilisation de l'unité de désinfection (5) et transmission du signal de déclenchement à l'unité de commande,
prendre en compte le signal de déclenchement par l'unité de commande lors de la commande de la période de fonctionnement de l'appareil (1).

10. Procédé selon la revendication 9, **caractérisé en ce qu'**un déclenchement de l'exécution du programme de nettoyage par l'unité de commande s'effectue dès qu'un signal de déclenchement a été transmis après la fermeture du volet (4).

11. Procédé selon la revendication 9 ou 10, **caractérisé en ce qu'**un déclenchement du volet (4) est effectué dès qu'un signal de déclenchement a été transmis après la fin du programme de nettoyage.
